(19) **Europäisches Patentamt**
**European Patent Office**
**Office européen des brevets**

(11) **EP 3 849 407 B1**

(12) # EUROPEAN PATENT SPECIFICATION

(45) Date of publication and mention
of the grant of the patent:
**31.01.2024 Bulletin 2024/05**

(21) Application number: **19859532.4**

(22) Date of filing: **12.09.2019**

(51) International Patent Classification (IPC):
*A61B 5/0205* (2006.01)   *A61B 5/08* (2006.01)
*A61B 5/1455* (2006.01)   *A61B 5/00* (2006.01)
*A61B 5/113* (2006.01)   *G16H 40/67* (2018.01)
*G16H 50/20* (2018.01)

(52) Cooperative Patent Classification (CPC):
**A61B 5/681; A61B 5/0205; A61B 5/0816;
A61B 5/14551; A61B 5/4842; A61B 5/7235;
A61B 5/7275; G16H 40/67; G16H 50/20;**
A61B 5/113; A61B 2562/0219

(86) International application number:
**PCT/IL2019/051018**

(87) International publication number:
**WO 2020/053858 (19.03.2020 Gazette 2020/12)**

(54) **SYSTEM AND METHOD FOR MONITORING RESPIRATORY RATE AND OXYGEN SATURATION**

SYSTEM UND VERFAHREN ZUR ÜBERWACHUNG DER ATEMFREQUENZ UND DER
SAUERSTOFFSÄTTIGUNG

SYSTÈME ET PROCÉDÉ DE SURVEILLANCE DE LA FRÉQUENCE RESPIRATOIRE ET DE LA
SATURATION EN OXYGÈNE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.09.2018 US 201816132224**

(43) Date of publication of application:
**21.07.2021 Bulletin 2021/29**

(60) Divisional application:
**23162749.8 / 4 218 558**

(73) Proprietor: **Chronisense Medical Ltd.
2069202 Yokneam (IL)**

(72) Inventor: **LANGE, Daniel H
2069202 Yokneam (IL)**

(74) Representative: **Weal, Emily Teresa et al
Keltie LLP
No.1 London Bridge
London SE1 9BA (GB)**

(56) References cited:
WO-A1-2013/061415   WO-A1-2016/110804
WO-A1-2017/079828   WO-A1-2017/140696
US-A1- 2010 179 438   US-A1- 2011 257 551
US-A1- 2016 007 935   US-A1- 2017 156 593
US-A1- 2018 132 794   US-A1- 2018 132 794
US-A1- 2019 015 014

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

CROSS-REFERENCE TO RELATED APPLICATIONS

The present application is a Continuation-in-Part of U.S. Patent Application No. 14/738,666, titled "Monitoring Health Status of People Suffering from Chronic Diseases," filed on June 12, 2015, a Continuation-in-Part of U.S. Patent application No. 14/738,636, titled "Wearable Device Electrocardiogram," filed on June 12, 2015, a Continuation-in-Part of U.S. Patent Application No. 14/738,711, titled "Pulse Oximetry," filed on June 12, 2015, and a Continuation-in-Part of U.S. Patent Application No. 14/983,118, titled "Using Invariant Factors for Pulse Oximetry," filed on December 29, 2015. U.S. Patent Application No. 14/983,118 is a Continuation-in-Part of U.S. Patent Application No. 14/738,666, titled "Monitoring Health Status of People Suffering from Chronic Diseases," filed on June 12, 2015, a Continuation-in-Part of U.S. Patent application No. 14/738,636, titled "Wearable Device Electrocardiogram," filed on June 12, 2015, and a Continuation-in-Part of U.S. Patent Application No. 14/738,711, titled "Pulse Oximetry," filed on June 12, 2015.

FIELD

The present application relates to systems and methods for monitoring medical parameters of people, and more specifically to systems and methods for monitoring respiratory rate and oxygen saturation.

BACKGROUND

It should not be assumed that any of the approaches described in this section qualify as prior art merely by virtue of their inclusion in this section.

Monitoring chronic diseases, which includes measuring medical parameters, is central to providing appropriate and timely treatment to patients suffering from such chronic diseases as chronic heart failure, cardiac arrhythmia, chronic obstructive pulmonary disease, asthma, and diabetes. Traditionally, monitoring is carried out and measurements are taken while a patient is hospitalized or in other clinical settings. Appropriate treatment regimens can be based on these measurements, and thus it is highly beneficial to monitor medical parameters of the patient after the patient is released from the hospital. Therefore, the patient can be asked to visit the hospital or clinic periodically for monitoring and adjustment of treatment, if necessary.

However, most often, no measurements are carried out between visits, usually due to the need for trained examiners and medical devices. This is unfortunate, because, between visits, the chronic disease from which the patient suffers can worsen and result in emergency treatment and hospitalization. Furthermore, after receiving repeated courses of emergency hospital treatment, the patient's health condition may degrade and never return to the pre-hospitalization level. Therefore, a technology that allows for at-home measurements of medical parameters can be essential to managing chronic diseases or even saving a patient's life. Early warnings of worsening conditions associated with chronic diseases may prevent unnecessary hospitalizations by providing a preventive treatment and, as a result, reduce financial and human costs of the hospitalization and treatment.

International Patent Publication No. WO 2017/079828 A1 discloses an apparatus for measuring cardiopulmonary data of a wearer, comprising: a sensor operable to produce a data stream indicative of movements of a wearer's body; a positioning device holding said sensor proximate an anatomical landmark on said wearer's body for conduction of mechanical vibrations from said wearer's body to said sensor; and a processor configured to receive said data stream and produce a rate signal indicative of cardiac or respiratory rate data of said wearer using an algorithm comprising peak detection.

International Patent Publication No. WO 2013/061415 A1 discloses measuring respiratory rate with sensing data from a wristband sensor node. The respiration measurement system is provided with: a sensor that is worn on the arm and acquires sensing data representing movements of the arm; a main cyclic component-detecting unit that periodically tabulates sensing data for a prescribed elapsed period and detects the main cyclic component thereof; a respiratory information acquisition validity-assessing unit that assesses whether or not said main cyclic component is valid as a respiration-dependent cyclic component on the basis of the frequency and/or amplitude of the main cyclic component according to previously determined criteria; and a storage unit that stores the frequency of the main cyclic component as a respiratory rate correlated with time information when the main cyclic component is determined to be valid as a respiration-dependent cyclic component.

US Patent Publication No. US 2016/007935 A1 discloses a sensor system that includes one or more gyroscopes and one or more accelerometers, for measuring subtle motions of a user's body. The system estimates physiological parameters of a user, such as heart rate, breathing rate and heart rate variability. When making the estimates, different weights are assigned to data from different sensors. For at least one estimate, weight assigned to data from at least one gyroscope is different than weight assigned to data from at least one accelerometer. Also, for at least one estimate, a weight assigned to one or more sensors located in a first region relative to the user's body is different than a weight assigned to one or more sensors located in a second region relative to the user's body. Furthermore, weight assigned to data from at least one sensor changes over time.

International Patent Publication No. WO 2017/140696 A1 discloses a device comprising a signal input (21) for obtaining sensor signals representing orientation changes and/or motion of a sensor, which is configured to measure orientation changes and/or motion of the sensor and to generate said sensor signals. The device comprises a selector for determining combination values of said sensor signals and for selecting one or more of said sensor signals either having the most extreme combination values or that most likely coincide with the direction of a breathing motion, which direction is determined from said combination values, a converter for converting the selected sensor signals into the frequency domain to obtain one or more frequency-domain sensor signals, and a peak detector for performing maximum peak detection in said frequency-domain sensor signals within a predetermined frequency range and selecting the frequency corresponding to the detected maximum peak as breathing rate.

US Patent Publication No. US 2018/132794 A1 discloses determining an early warning score for a health status of a patient. An example method includes acquiring, during a predetermined time period via sensors integrated into a wearable device worn on a wrist of the patient, initial values of medical parameters of patient. The medical parameters include a respiratory rate, oxygen saturation, temperature, blood pressure, and pulse rate, and level of consciousness. The method includes determining, based on the initial values, normal values of the medical parameters. The method includes acquiring, via the sensors and at a pre-determined frequency, further values of the medical parameters. The method includes determining, based on deviations of the further values from the normal values, individual scores for the medical parameters. The method includes calculating, based on the individual scores, a general score. The method includes determining, based on the general score, the health status of the patient.

SUMMARY

This summary is provided to introduce a selection of concepts in a simplified form that are further described below in the Detailed Description. This summary is not intended to identify key features or essential features of the claimed subject matter, nor is it intended to be used as an aid in determining the scope of the claimed subject matter. The invention is defined in the appended claims.

According to one embodiment of the present disclosure, a system for monitoring medical parameters of a patient is provided. The system includes a wearable device configured to be disposed around a wrist of the patient. The wearable device includes a gyroscope configured to provide a gyroscope signal indicative of a motion of the patient. The system further includes a processor communicatively coupled to the gyroscope. The processor is configured to perform a spectral analysis of the gyroscope signal to obtain a spectrum in a pre-determined range. The pre-determined range covers a normal respiratory rate range. The processor is configured to determine a position of a peak in the spectrum to obtain a value for the respiratory rate. The processor is configured to provide, based on the value of the respiratory rate, a message regarding a health status of the patient.

Based on the determination that the value of the respiratory rate is outside the normal respiratory range, the processor can provide an alert message regarding the health status of the patient. The spectral analysis can be performed by a method of averaged periodograms. A normal respiratory rate range may include 6 to 18 breaths per minute.

The processor is configured to determine the strongest amplitude peak in the spectrum to obtain a value for the respiratory rate. The processor determines that the spectrum includes one or more further peaks with descending amplitudes. The further peaks correspond to frequencies $n*\omega$, wherein $\omega$ is a frequency corresponding to the strongest amplitude peak, and n is a natural number. Based on the determination, the processor assigns $\omega$ to the value of the respiratory rate.

The wearable device may further include a first optical sensor configured to measure, at a palmar surface of the wrist, a first red wavelength photoplethysmography (PPG) signal and a first infrared wavelength PPG signal. The wearable device may further include a second optical sensor configured to measure, at a dorsal surface of the wrist, a second red wavelength PPG signal and a second infrared wavelength PPG signal. The first optical sensor and the second optical sensor can be communicatively coupled to the processor.

The processor can be configured to determine, based on the first red wavelength PPG signal and the first infrared wavelength PPG signal, a first ratio for obtaining an oxygen saturation. The processor can be further configured to determine, based on the second red wavelength PPG signal and the second infrared wavelength PPG signal, a second ratio for obtaining the oxygen saturation. The processor can be further configured to determine, based on the first ratio and the second ratio, a third ratio to be used for obtaining an oxygen saturation. The processor can be further configured to determine, based on the third ratio, a value of the oxygen saturation. The processor can be further configured to provide, based on the value of the oxygen saturation, a message regarding a health status of the patient.

The third ratio can be determined by formula $R = \alpha R_a + (1 - \alpha)R_b$, wherein the $\alpha$ is a weight between 0 and 1, the $R_a$ is the first ratio, and the $R_b$ is the second ratio. The weight $\alpha$ can be a function of a perfusion index. The perfusion index can be determined based on the second red wavelength PPG signal or the second infrared wavelength PPG signal. The weight $\alpha$ increases when the perfusion index increases. A shape of the function is pre-determined during a calibration process.

The first optical sensor can be configured to measure the first red wavelength PPG signal and the first infrared wavelength PPG signal substantially near a radial artery of the wrist.

According to another example embodiment of the present disclosure, a method for monitoring medical parameters of a patient is provided, as defined in appended Claim 3.

The method can include measuring at a palmar surface of the wrist of the patient, by a first optical sensor of the wearable device, a first red wavelength PPG signal and a first infrared wavelength PPG signal. The method may include measuring at a dorsal surface of the wrist, by a second optical sensor of the wearable device, a second red wavelength PPG signal and a second infrared wavelength PPG signal. The first optical sensor and the second optical sensor can be communicatively connected to the processor.

The method may further include determining, by a processor and based on the first red wavelength PPG signal and the first infrared wavelength PPG signal, a first ratio for obtaining an oxygen saturation.

The method may include determining, by the processor and based on the second red wavelength PPG signal and the second infrared wavelength PPG signal, a second ratio to be used for obtaining the oxygen saturation. The method may further include determining, by the processor and based on the first ratio and the second ratio, a third ratio for obtaining an oxygen saturation. The method may further include determining, by the processor and based on the third ratio, a value of the oxygen saturation. The method may further include providing, by the processor based on the value of the oxygen saturation, a message regarding a health status of the patient.

According to another example embodiment of the present disclosure, the steps of the method for monitoring medical parameters of a patient are stored on a non-transitory machine-readable medium comprising instructions, which when implemented by one or more processors perform the recited steps.

Other example embodiments of the disclosure and aspects will become apparent from the following description taken in conjunction with the following drawings.

BRIEF DESCRIPTION OF THE DRAWINGS

Embodiments are illustrated by way of example and not limitation in the figures of the accompanying drawings, in which like references indicate similar elements and in which:

FIG. 1 is a block diagram showing an example system for monitoring medical parameters of a patient.
FIG. 2 is a block diagram showing components of an example device for monitoring medical parameters of a patient.
FIG. 3 is a block diagram illustrating example sensors, example medical parameters, and example chronic diseases.
FIGs. 4A and 4B are schematic diagrams illustrating an example device for monitoring medical parameters of a patient.
FIG. 4C is a schematic diagram illustrating an example optical sensor.
FIG. 5 is a block diagram showing an example system for monitoring medical parameters of a patient.
FIG. 6A is a plot of an example PPG signal.
FIG. 6B shows an example function 600 for determining the weight $\alpha$ based on the value of a perfusion index.
FIG. 7 shows plots of a gyroscope signal, spectrum of the gyroscope signal, reference signal, and spectrum of the reference signal.
FIG. 8 shows plots of another gyroscope signal, spectrum of another gyroscope signal, another reference gyroscope signal, and spectrum of another reference gyroscope signal.
FIG. 9 is a flow chart showing steps of an example method for monitoring a respiratory rate.
FIG. 10 is a flow chart diagram illustrating an example method for monitoring respiratory rate of a patient.

DETAILED DESCRIPTION

The following detailed description includes references to the accompanying drawings, which form a part of the detailed description. The drawings show illustrations in accordance with exemplary embodiments. These exemplary embodiments, which are also referred to herein as "examples," are described in enough detail to enable those skilled in the art to practice the present subject matter. The embodiments can be combined, other embodiments can be utilized, or structural, logical and electrical changes can be made without departing from the scope of what is claimed. The following detailed description is, therefore, not to be taken in a limiting sense, and the scope is defined by the appended claims.

The present disclosure provides systems and methods for monitoring medical parameters of people suffering from chronic diseases. Embodiments of the present disclosure can allow measuring medical parameters of a patient in a non-intrusive manner while, for example, the patient is at home, at work, outdoors, traveling, and at other stationary or mobile environments. Some example embodiments can provide for a wearable device (e.g., a wristband, a watch, or a bracelet) that includes sensors configured to measure medical parameters such as, for example, oxygen saturation, respiratory rate, and the like. The measurements can be taken during daytime and nighttime for days, weeks, months, and years. The medical parameters can be analyzed to determine trends in the medical parameters and to determine whether the severity of the patient's chronic disease (e.g., a heart disease, diabetes, lung disease, and so on) worsens or improves. Embodiments of the present technology may facilitate a rapid reaction to provide an appropriate and timely treatment for the patient. The early treatment may allow taking timely preventive measures to avoid worsening of the patient's condition to the point of requiring an emergency hospitalization and associated expensive medical treatment.

According to various example embodiments, a method for monitoring medical parameters of a patient can include measuring at a palmar surface of a wrist of the patient, by a first optical sensor of a wearable device configured to be disposed around the wrist of the patient, a first red wavelength PPG signal and a first infrared wavelength PPG signal. The method may include measuring from a dorsal surface of the wrist, by a second optical sensor, a second red wavelength PPG signal and a second infrared wavelength PPG signal. The method may also include determining, by a processor communicatively connected to the first optical sensor and the second optical sensor and based on the first red wavelength PPG signal and the first infrared wavelength PPG signal, a first ratio for obtaining an oxygen saturation.

The method may include determining, by the processor and based on the second red wavelength PPG signal and the second infrared wavelength PPG signal, a second ratio to be used to obtain the oxygen saturation. The method may further include determining, by the processor and based on the first ratio and the second ratio, a third ratio to be used to obtain an oxygen saturation. The method may further include determining, by the processor and based on the third ratio, a value of the oxygen saturation. The method may further include providing, by the processor based on the value of the oxygen saturation, a message regarding a health status of the patient.

The method may further include providing, by a gyroscope communicatively coupled with the processor and disposed in the wearable device, a gyroscope signal indicative of motion of the patient. The method may further include performing, by the processor, a spectral analysis of the gyroscope signal to obtain a spectrum in a pre-determined range. The pre-determined range may cover a normal respiratory rate range. The method may further include determining, by the processor, a position of a peak in the spectrum to obtain a value for a respiratory rate. The method may further include determining, by the processor, that the value of the respiratory rate is outside of the normal respiratory rate range. If the value of the respiratory rate is outside of the normal respiratory rate range, the method may further include providing, by the processor, an alert message regarding the health status of the patient.

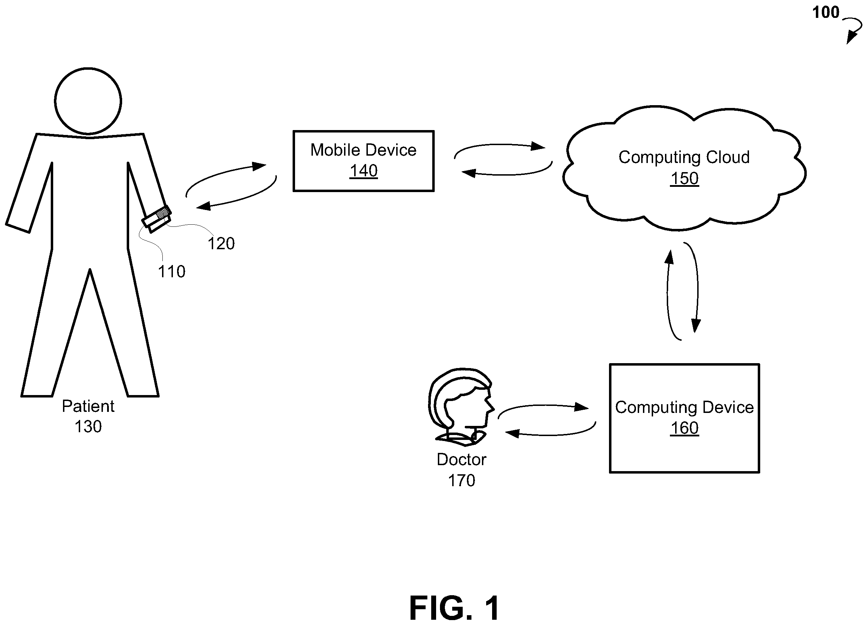

Referring now to FIG. 1, an example system 100 for monitoring a patient's health status is shown. The system 100 includes at least a wearable device 110. The wearable device may include sensors 120. In some embodiments, the wearable device 110 can be worn by a patient 130, for example on a wrist, for an extended period of time. The wearable device 110 can be carried out as a watch, a bracelet, a wristband, and the like.

The wearable device 110 can be configured to constantly collect, via sensors 120, sensor data from a patient 130. In some embodiments, based on the sensor data, a processor of the wearable device 110 can be configured to obtain, based on the sensor data, medical parameters associated with the patient 130. The medical parameters can be analyzed to obtain changes (trends) in medical parameters over time. Based on the changes, one or more conclusions regarding severity of one or more chronic diseases can be obtained. The processor of the wearable device 110 can be configured to send, via a communication module of the wearable device 110, messages regarding a current health status to a computing device of the patient, a relative, a caretaker of the patient, or a doctor treating the patient. The message to the patient may include an advice to see a doctor and/or take medicine. The processor of the wearable device 110 can display the message on a graphical display system of the wearable device 110.

In some embodiments, the system 100 includes a mobile device 140. The mobile device 140 can be communicatively coupled to the wearable device 110. In various embodiments, the mobile device 140 is operable to communicate with the wearable device 110 via a wireless connection such as, for example, Wi-Fi, Bluetooth, Infrared (IR), and the like. The mobile device 140 can include a mobile phone, a smart phone, a phablet, a tablet computer, a notebook, and so forth. The mobile device 140 can be configured to receive the sensor data and medical parameters from the mobile device 110. In certain embodiments, a processor of the mobile device can be configured to perform analysis of the received sensor data to determine medical parameters. The mobile device 140 can be further configured to provide, via a graphic display system of the mobile device 140, a report regarding current health status. In various embodiments, the mobile device 140 runs one or more applications that provide, via the graphical display system of the mobile device 140, charts and graphics concerning medical parameters of the patient.

In some embodiments, the mobile device 140 can be configured to determine the severity of a health status resulting from the chronic disease from which the patient suffers. The mobile device can be configured to provide the patient with advice to see a medical professional or to take medicine. An alert message regarding health status of the patient can be sent, by the mobile device 140, to a computing device of a doctor, relative, or caretaker of the patient.

In further embodiments, the system 100 may further include a cloud-based computing resource 150 (also referred to as a computing cloud). In some embodiments, the cloud-based computing resource 150 includes one or more server farms/clusters comprising a collection of computer servers and is co-located with network switches and/or routers. In certain embodiments, the mobile device 140 and/or wearable device 110 can be communicatively coupled to the computing cloud 150. The mobile device 140 and/or wearable device 110 can be operable to send the sensor data and medical parameters to the computing cloud 150 for further analysis. The computing cloud 150 can be configured to store historical data concerning patient health status including sensor data and medical parameters collected over days, weeks, months, and years. The computing cloud 150 can be configured to run one or more applications to provide reports regarding health status of the patient. A doctor 170 treating the patient may access the reports, for example via a computing device 160, using the Internet or a secure network. In some embodiments, the results of the analysis of the medical parameters can be sent back to the mobile device 140 and/or wearable device 110.

The severity of the health status resulting from a chronic disease can be estimated by computing a deviation or divergence from normal medical parameters of one or more medical parameters being measured at the moment. The normal medical parameters can be specific to the patient and can be derived based on historical data concerning the patient's health status recorded over an extended time period. If the deviation in the medical parameters becomes sufficiently large, the patient can be advised, via a message to the mobile device 140, to take medicine or contact a doctor. In some situations, when the deviation becomes substantial, an alert message can be sent by the mobile device 140 and/or the wearable device 110 to a computing device of a relative, a doctor, or a caretaker of the patient.

It may be desirable for the patient to be assured that the current medical parameters are within an acceptable deviation of the normal medical parameters. For example, when the current medical parameters are normal, the wearable device 110 and/or mobile device 140 can be operable to periodically alert the patient using a pleasant sound. The signal can be provided, for example, every 30 minutes, once every hour, and the like. In certain embodiments, when the medical parameters are within normal values, the mobile device 140 may provide a text message assuring the patient of normal conditions. A haptic feedback component can be used to alert the patient to a health condition, to warn the patient about a specific event concerning treatment of a chronic disease, to remind the patient to take a medicine, if the patient has failed to take the medicine within a pre-determined period of time, and so forth. The wearable device 110 may include a haptic feedback functionality for providing the patient with haptic feedback, for example, a tap-in device, to apply a force or vibration to skin of the patient. In further embodiments, the haptic alert can be provided by the mobile device 140. The mobile device 140 can vibrate when the mobile device is in a pocket of the patient or when the mobile device 140 is located on a surface (e.g., a table).

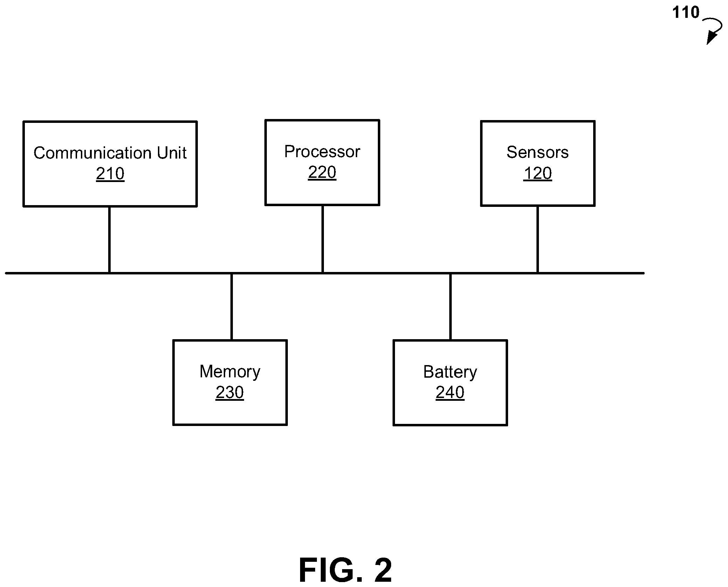

FIG. 2 is a block diagram illustrating components of wearable device 110, according to an example embodiment. The example wearable device 110 may include sensors 120, a communication unit 210, a processor 220, memory storage 230, and a battery 240.

The communication unit 210 can be configured to communicate with a network such as the Internet, a Wide Area Network (WAN), a Local Area Network (LAN), a cellular network, and so forth, to send a data stream, for example sensor data, medical parameters, and messages concerning the health condition of a patient.

The processor 220 can include hardware and/or software, which is operable to execute computer programs stored in memory 230. The processor 220 can use floating point operations, complex operations, and other operations, including processing and analyzing sensor data.

In some embodiments, the battery 240 is operable to provide electrical power for operation of other components of the wearable device 110. In some embodiments, the battery 240 is a rechargeable battery. In certain embodiments, the battery 240 is recharged using inductive charging technology.

The wearable device 110 may include additional or different components to provide a particular operation or functionality. Similarly, in other embodiments, the wearable device 110 includes fewer components that perform similar or equivalent functions to those depicted in FIG. 2. For example, the wearable device 110 may further include a graphical display system to provide an alert message and display current values of the medical parameters to the patient. The wearable device 110 may also include a haptic device to alert the patient with vibrations. The wearable device 110 may also include an audio signaling device, for example, a beeper or a speaker, to provide sound alerts to the patient.

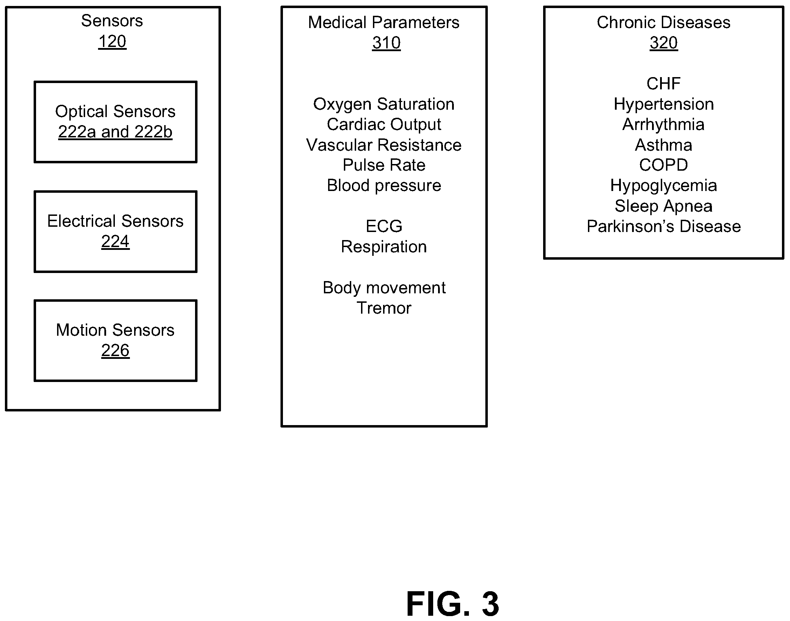

FIG. 3 is a block diagram showing a list of example sensors 120, a list of example medical parameters 310, and a list of example chronic diseases 320. In various embodiments, the sensors 120 may include optical sensors 222a and 222b, electrical sensors 224, and motion sensors 226. The medical parameters 310, determined based on the sensor data, include, but are not limited to, $SpO_2$ oxygen saturation, tissue oxygen saturation, cardiac output, vascular resistance, pulse rate, blood pressure, respiration, electrocardiogram (ECG) data, respiratory rate, and motion data. The chronic diseases 320, the progression of which can be tracked based on changes of the medical parameters, include but are not limited to congestive heart failure (CHF), hypertension, arrhythmia, asthma, chronic obstructive pulmonary disease (COPD), hypoglycemia, sleep apnea, and Parkinson's disease.

The optical sensors 222a and 222b can be operable to measure medical parameters associated with blood flow in blood vessels using changing absorbance of light at different wavelengths in blood and skin. The data from optical sensors 222a and 222b can be used to determine multiple medical parameters, including but not limited to: $SpO_2$ oxygen

saturation, cardiac output, vascular resistance, pulse rate, and respiratory rate. Based on the measurements obtained from optical sensors 222a and 222b, abnormal cardiac rhythms (for example, atrial fibrillation, rapid rhythms, and slow rhythms) can be detected.

In some embodiments, respiratory rate can be derived from a sinus arrhythmia waveform. The sinus arrhythmia waveform can be obtained based on intervals between subsequent heart beats (RR intervals) measured by the optical sensors 222a and 222b using the fact that the rhythm of the heart beats is modulated by human breathing.

The electrical sensors 224 can be operable to obtain ECG activity data of the patient. The ECG activity data includes a characteristic electrically-derived waveform of a heart activity. The ECG data can include a number of components, whose characteristics (timing, amplitude, width, and so forth), alone or in combination, can provide a picture of cardiac and overall health. The ECG data is typically derived by measurements from one or more sets of leads (groups of electrodes comprising grounded circuits), such that the exact characteristics of the resulting waveforms is a function of the electrical and spatial vectors of the electrode positions relative to the heart. While the details of interpretation of the ECG data are too involved to describe succinctly in this disclosure, consideration of each of the component parameters can indicate health status, physical or psychological stress, or trends of disease conditions. Various cardiovascular parameters can be extracted from the ECG alone (such as a heart rate for example), or in combination with other physiological measurements.

According to example embodiments of present disclosure, ECG of the patient can be measured via the electrical sensors 224. Since measurements are taken from a wrist of the patient, electrodes of the electrical sensors 224 should be located very close to each other on a wearable device. Therefore, the ECG data may contain noise. Averaging of several subsequent intervals of the ECG data between heart beats can be used to cancel out noise in ECG data. To determine intervals between two subsequent heart beats, the PPG signals as measured by optical sensors 222a and 222b can be used as a reference. In some embodiments, an arrhythmia analysis can be carried out using the ECG data and data concerning cardiac output and pulse rate.

In some embodiments, motion sensors 226 include an accelerometer, a gyroscope, and an Inertial Measurement Unit (IMU). The motion data obtained via motion sensors 226 can provide parameters of body movement and tremor. The motion data can allow tracking the progression or remission of a motor disease, Parkinson's disease, and physical condition of the patient. In some embodiments, the motion data can be analyzed to determine whether the patient is likely to fall. In some embodiments, the motion data can be analyzed in time domain and frequency domain. By tracking amplitudes of movement of the patient it can be determined if the patient's movements become slower (i.e., the patient becomes sluggish) or the patient is not moving at all. Additionally, according to example embodiments of the present disclosure, the motion data can be analyzed to determine a respiratory rate of the patient.

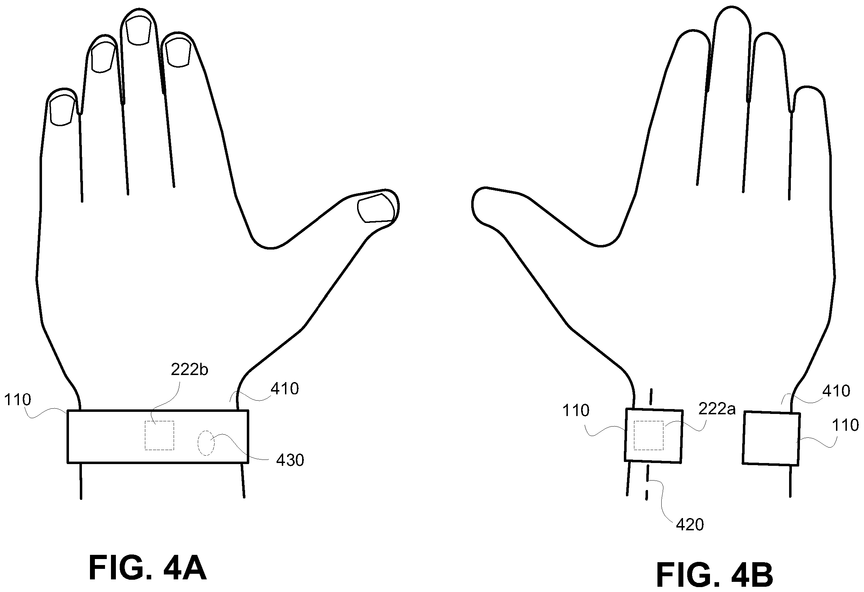

FIG. 4A and FIG. 4B are schematic diagrams illustrating an example wearable device 110. In the examples of FIG. 4A and FIG 4B, the wearable device 110 can be carried out in a shape of an open bangle. The FIG. 4A shows a dorsal side of a patient's hand 410 and the wearable device 110 placed on the patient's wrist. FIG. 4B shows a palmar side of the patient's hand 410 and wearable device 110. The wearable device 110 can be designed to apply pressure at an area of skin surface covering a radial artery 420. In comparison to wristbands and straps, an open bangle may be more comfortable to wear by a patient since no pressure is applied to the middle area inside the wrist.

The wearable device 110 can include optical sensors 222a and 222b located on an inner side of the wearable device 110. When the wearable device 110 is worn on the patient's hand, the inner side of the wearable device 110 can be in continuous contact with a surface of the skin of the patient's hand 410.

When the wearable device 110 is disposed around a wrist of patient's hand 410, the sensor 222a is located as close as possible to cover the skin area covering an artery of the wrist, for example the radial artery 420. When the wearable device 110 is disposed around a wrist of patient's hand 410, the optical sensor 222b is located on the dorsal side of the wrist. The sensors 222a and 222b can be configured to be in a continuous contact with the skin of the patient 110.

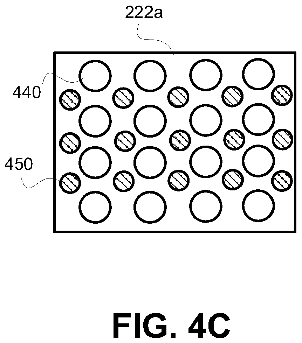

As shown in FIG. 4C, the optical sensors 222a may include multiple light sensors 440 (photodetectors), to measure the reflected light, and multiple light transmitters 450 (for example, Light Emission Diodes (LEDs)). The number and location of the light sensors 440 and light transmitters 450 can be chosen such that in case of an accidental displacement of the wearable device, at least one of the light sensors is still located sufficiently close to the radial artery. In some embodiments, when measuring the light reflected from the skin and radial artery, a signal from those photoelectric cells that provides the strongest or otherwise determined best output can be selected for further processing in order to obtain medical parameters using methods of pulse (reflectance) oximetry. In certain embodiments, the wearable device 110 can be configured to apply a pre-determined amount of pressure to the wrist each time the user wears the wearable device to allow the same conditions for the reflection of the light from the skin. The optical sensor 222b may include elements analogous to the elements of the optical sensor 222a. The signals measured by optical sensors 222a and 222b can be used to determine oxygen saturation, heart rate, cardiac output, and other parameters using pulse oximetry methods.

Oxygen saturation is the relative proportion (typically expressed as percentage) of oxygen dissolved in blood, as bound to hemoglobin, relative to non-oxygen-bound hemoglobin. Oxygen saturation is important in clinical monitoring of surgical anesthesia, and in monitoring and assessment of various clinical conditions such as COPD and asthma. In healthy individuals, oxygen saturation is over 95%. Direct measurement can be made from arterial blood sample, but drawing blood is an invasive procedure, and, except for a few controlled environments (e.g., during a surgery) cannot be easily performed continuously. Pulse oximetry can yield a quantity called SpO2 (saturation of peripheral oxygen), an accepted estimate of arterial oxygen saturation, derived from optical characteristics of blood using transmission of light through a thin part of a human body (for example, a fingertip or an earlobe (in the most common transmissive application mode)). Reflectance pulse oximetry can be used to estimate SpO2 using other body sites. The reflectance pulse oximetry does not require a thin section of the person's body and is therefore suited to more universal application such as the feet, forehead, and chest, but it has some serious issues due to the light reflected from non-pulsating tissues. When oxygen saturation cannot be measured directly from arterial blood, an indirect measurement can be performed by tracking tissue oxygen saturation. The measurement of oxygen saturation is commonly used to track progression of heart disease or lung disease. When the heart or lungs are not functioning properly, the saturation of oxygen drops in both arterial blood and tissue around the artery. Therefore, tissue oxygen saturation can be measured by sensing the skin color near the radial artery. For example, if the wearable device 110 moves so that sensor 222a is not covering the radial artery, measurements of tissue saturation near the radial artery can be used as a backup to provide values for oxygen saturation.

The optical sensor 222a can be configured to measure an infrared wavelength PPG signal $I_a^{ir}$ and a red wavelength PPG signal $I_a^{red}$ near a blood artery of the wrist of the patient (for example, the radial artery 420). The infrared wavelength PPG signal can be obtained by emitting, by the light transmitters 450, an infrared wavelength light and detecting, by the light sensor 440, intensity of infrared light reflected from the skin and blood vessels of the patient. The red wavelength PPG signal can be obtained by emitting, by the light transmitters 450, a red wavelength light and detecting, by the light sensor 440, intensity of red light reflected from the skin and blood vessels of the patient. Similarly, the optical sensor 222a can be configured to measure an infrared wavelength PPG signal $I_b^{ir}$ and a red wavelength PPG signal $I_b^{red}$ from the palmar side of the wrist of patient.

The wearable device 110 includes a gyroscope 430. The gyroscope 430 can be located at any point within the wearable device 110. The gyroscope may include a triple axis Micro Electro-Mechanical Systems (MEMS) gyroscope. The gyroscope 430 can measure rotation around three axes: x, y, and z.

In some embodiments, a gyroscope signal measured by the gyroscope 430 is analyzed to determine a respiratory rate of a patient. The respiratory rate, which is a vital sign, is typically expressed as the number of breaths per minute. Typical adult resting respiratory rate is about 16-20 breaths per minute. Extreme variations can result from physical or psychological stress. The respiratory rate is often affected in chronic disease states, particularly in pulmonary and cardiovascular disease. Extreme short-term changes may be associated with acute disease episodes, particularly in chronically ill patients.

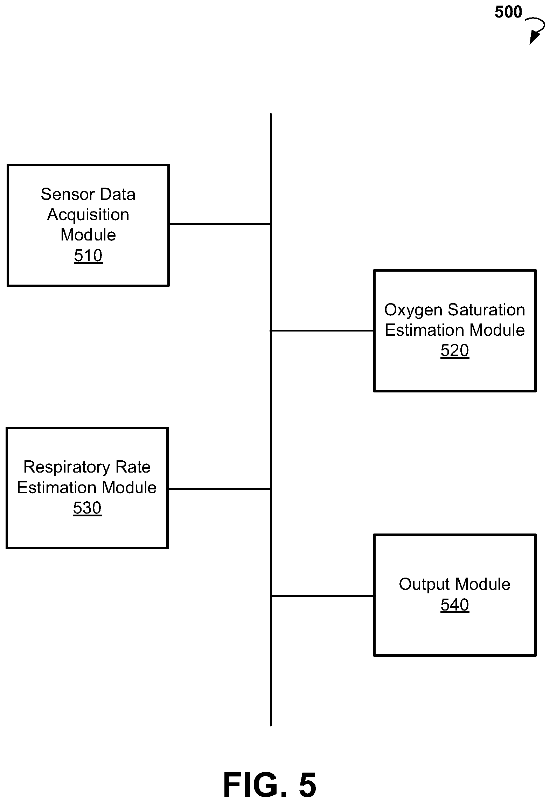

FIG. 5 is a block diagram showing components of a system 500 for monitoring health status of people suffering from chronic diseases, according to an example embodiment. The system 500 can include a sensor data acquisition module 510, an oxygen saturation estimation module 520, a respiratory rate estimation module 530, and an output module 540. In some embodiments, the modules 510-540 are implemented as chipsets included in the wearable device 110. In other embodiments, the modules 520, 530, and 540 can be stored as instructions in memory of the wearable device 110, mobile device 140, or computing cloud 150, and executed by a processor.

In some embodiments, the sensor acquisition module 510 is configured to receive and digitize the sensor data. The sensor acquisition module 510 can include one or more analog-to-digital converters to transform the electrical signals from sensors 120 to digital signals and provide the digital signal to modules 520-540 for further analysis.

In some embodiments, the data processing module is configured to analyze the infrared wavelength PPG signals and the red wavelength PPG signals recorded by the optical sensor 222a and 222b to obtain oxygen saturation using the methods of pulse oximetry.

The methods for pulse oximetry are based on the fact that oxygenated hemoglobin absorbs more infrared light while deoxygenated hemoglobin absorbs more red light. Typically, the methods of the pulse oximetry include calculation of a ratio $R$ defined as follows:

$$R = \frac{log\left(\frac{I^{red}_{max}}{I^{red}_{min}}\right)}{log\left(\frac{I^{ir}_{max}}{I^{ir}_{min}}\right)} \qquad (1)$$

wherein $I^{red}_{max}$ and $I^{red}_{min}$ are maximum and minimum of red wavelength PPG signal and $I^{ir}_{max}$ and $I^{ir}_{min}$ are maximum and minimum of infrared wavelength PPG signal. The ratio R can indicate a ratio between oxygenated hemoglobin and deoxygenated hemoglobin. The ratio $R$ can be converted to a corresponding oxygen saturation ($SpO_2$) value via an empirically-derived look-up table.

In some embodiments, to take into account contributions due to interaction of light with non-pulsatile tissue, the infrared wavelength PPG signal can be shifted by a parameter $L^{ir}$ and the red wavelength PPG signal can be shifted by a parameter $L^{red}$. The ratio $R_1$ can be then determined as

$$R_1\left(L^{red}, L^{ir}\right) = \frac{log\left(\frac{I^{red}_{max} - L^{red}}{I^{red}_{max} - L^{red}}\right)}{log\left(\frac{I^{ir}_{max} - L^{ir}}{I^{ir}_{min} - L^{ir}}\right)} \qquad (2)$$

The ratio Ri can be further used to obtain the oxygen saturation via an empirically-derived look-up table or function. The parameters $L^{red}$ and $L^{ir}$ can be pre-determined in a calibration process. The calibration process may include optimization of $L^{red}$ and $L^{ir}$ to fulfill the equation $R_1(L^{red}, L^{ir}) = R_{true}$, wherein the $R_{true}$ is a true value for the ratio R found by a "gold standard" measurement.

Using the infrared wavelength PPG signal $I^{ir}_a$ and the red wavelength PPG signal $I^{red}_a$ recorded by the optical sensor 222a from palmar side of a wrist near a blood artery, the module 520 may be configured to calculate a ratio $R_a$. Using the infrared wavelength PPG signal $I^{ir}_b$ and red wavelength PPG signal $I^{red}_b$ recorded by the optical sensor 222b from dorsal side of the wrist, the module 520 may be configured to calculate a ratio $R_b$. The PPG signals $I^{ir}_a, I^{red}_a, I^{ir}_b$, and $I^{red}_b$ can be recorded substantially simultaneously.

Since the ratio $R_a$ is determined from PPG signals recorded by optical sensor 222a from the palmar side of the wrist near a blood artery, the value of the ratio $R_a$ may be sensitive to location of light transmitters 450 and light sensors 440 relative to the blood artery. Typically, error of value oxygen saturation ($SpO_2$) determined based on ratio $R_a$ decreases with increase of values for oxygen saturations. However, the sensor 222a located at the radial artery on the palmar side of the wrist may provide a PPG signals with a Signal-to-Noise Ratio (SNR) higher than the SNR of the PPG signal for sensor 222b located on the dorsal side of the wrist. Therefore, a location of an optical sensor near the radial artery is preferable over locations near fingertip or dorsal side of the wrist. Optical sensors located at a fingertip or on the dorsal side of the wrist can measure PPG signals received from small blood vessels. The quality of the PPG measurements received from the small blood vessels may be decreased due to several factors. One of the factors causing decrease in the quality of the PPG measurements includes possible constriction of small vessels due to low ambient temperatures. Another factor causing decrease in the quality of the PPG measurements includes a darker color of the skin covering the small blood vessels, which may lead to a decrease of the SNR in the PPG signals.

The ratio $R_b$ is determined from PPG signals recorded at dorsal side of the wrist, which does not include pulsatile blood vessels. Therefore, the ratio $R_b$ can be assumed to be independent on location of the light transmitters and light sensors of the optical sensor 222b relative to the surface of the dorsal side of the wrist. When PPG signals $I^{ir}_b$, and $I^{red}_b$ recorded by the optical sensor 222b are of a good quality, that is having a high SNR (as defined above), then the ratio $R_b$ obtained using the PPG signals $I_b^{ir}$, and $I_b^{red}$ can be used to calibrate the PPG signals $I_a^{ir}$ and $I_a^{red}$ recorded by the optical sensor 222a.

In some embodiments, the ratio $R_a$ can be found by using the formula (2) and the ratio $R_b$ can be found by using formula (1). The ratio $R_b$ can be further used as $R_{true}$ (the "gold standard" measurement) to optimize the parameters L$^{red}$ and L$^{ir}$ in formula (2). Thus, the PPG signals measured by the optical sensor 222b on the dorsal side of the wrist can be used to calibrate the optical sensor 222a on the palmar side. The calibration process can be carried out each time the patient wears the wearable device 110. The calibration process can be also performed if a value of the oxygen saturation determined by using the PPG signals measured by the optical sensor 222a is outside a pre-determined range.

In some embodiments, the ratio $R_b$ can be used to correct a value for ratio $R_a$. For example, the ratio $R_a$ and ratio $R_b$ can be fused to obtain a ratio R. The ratio R can be further used to determine value of the oxygen saturation via an empirically-derived look-up table.

In some embodiments, a ratio R can be determined as

$$R = \alpha R_a + (1 - \alpha)R_b \quad (3)$$

wherein $\alpha$ is a weight, $0 < \alpha \le 1$. The weight $\alpha$ can be determined as a function of SNR of PPG signal $I_b^{ir}$ or PPG signal $I_b^{red}$ measured at the palmar side of the wrist. Higher values of the weight $\alpha$ may correspond to higher values of the SNR of PPG signals $I_b^{ir}$, or $I_b^{red}$ since a higher SNR signal is more reliable for determining ratio R. A weighted average of the ratio $R_a$ and ratio $R_b$ can provide a better estimate for the ratio R because measurement errors of the sensor 222a and 222b are independent of each other. At the same time, the ratio $R_a$ can be given a larger weight because a measurement obtained on the palmar side of the wrist and near the radial artery may provide more information for core blood pressure and oxygen saturation than a measurement obtained from small vessels on the dorsal side of the wrist.

In some other embodiments, weight $\alpha$ can be determined as a function of a perfusion index. The perfusion index is a ratio of the pulsatile blood flow to the non-pulsatile static blood flow in patient's peripheral tissue. The perfusion index can be estimated based on a PPG signal $I_b^{ir}$ or a PPG signal $I_b^{red}$ measured at the dorsal side of the wrist.

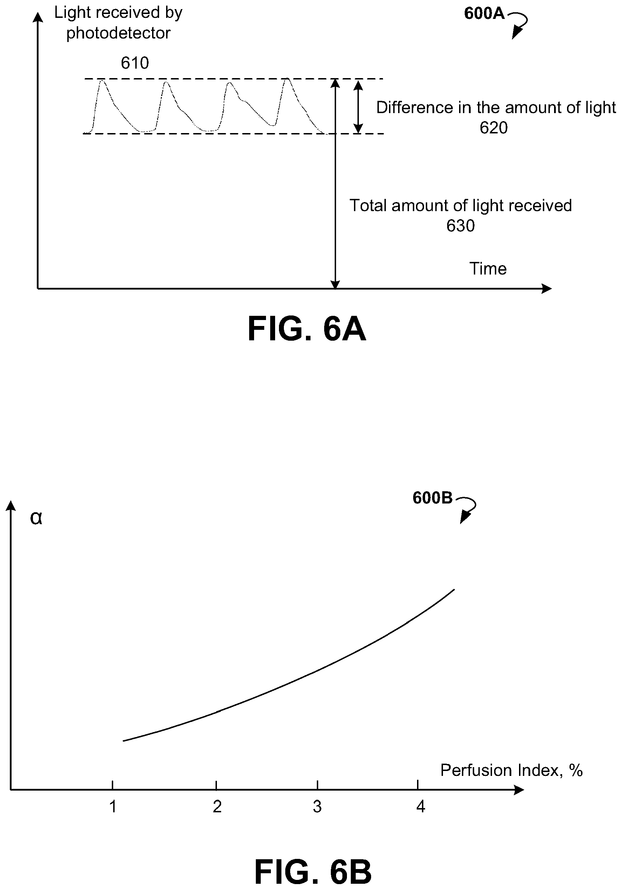

FIG. 6A shows a plot 600A of an example PPG signal 610 which represents an amount of light received by a photodetector of sensor 222b. The perfusion index can be calculated as a ratio of difference 620 of the amount of light received by the photodetector between vasoconstriction and vascular dilation to the total amount 630 of light received by the photodetector. Typically, the larger the perfusion index, the more accurate the measurement of oxygen saturation. Therefore, the perfusion index can be used as an indicator of quality of the PPG signal.

If the perfusion index determined based on PPG signals PPG signal $I_b^{ir}$ and PPG signal $I_b^{red}$ measured at the dorsal side of the wrist are higher than a pre-determined threshold, the ratio $R_b$ obtained using the PPG signals $I_b^{ir}$ and $I_b^{red}$ can be used to correct the ratio $R_a$ using formula (3). Furthermore, if the perfusion index determined based on the PPG signal $I_b^{ir}$ and PPG signal $I_b^{red}$ measured at the dorsal side of the wrist are higher than a pre-determined threshold, the ratio $R_b$ can be also used to determine parameters L$^{red}$ and L$^{ir}$ in formula (2) for shifting PPG signals $I_a^{red}$ and $I_a^{ir}$ and, thereby, calibrate the sensor 222a disposed at the palmar side of the wrist.

FIG. 6B shows an example function 600B for determining the weight $\alpha$ based on the value of perfusion index. The value of the perfusion index can be found based on the PPG signal $I_b^{ir}$ or the PPG signal $I_b^{red}$ measured at the dorsal side of wrist of a patient. The shape of function 600 can be determined in a calibration process. The weight $\alpha$ increases with an increase of the value for the perfusion index.

The respiratory rate estimation module 530 is configured to analyze the gyroscope signal (rotation around three axes x, y, and z) measured by the gyroscope 430 at a wrist to obtain respiratory rate of a patient. It was found in tests performed by inventor that the gyroscope signal measured at the wrist of the patient includes data due to a cyclical motion of the chest of the patient. During the tests, a gyroscope signal measured by a gyroscope disposed at a wrist of a patient and a reference gyroscope signal measured by a gyroscope disposed in a chest strap at the chest of the patient were recorded simultaneously. A spectral analysis of the gyroscope signal measured at the wrist and the reference gyroscope signal measured at the chest were performed. A comparison of the spectra of the signals shows that the gyroscope signal measured at the wrist contains a frequency corresponding to the number of breaths of patient as determined based on the reference gyroscope signal measured at the chest.

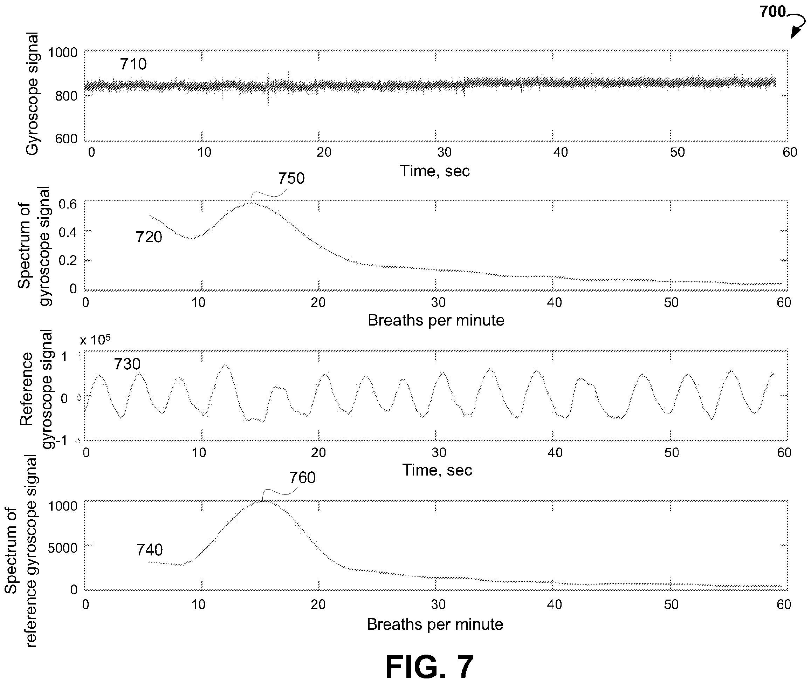

FIG. 7 shows plot 700 of example gyroscope signal 710 measured at a wrist, spectrum 720 of the gyroscope signal 710, reference gyroscope signal 730 measured at the chest, and spectrum 740 of the reference gyroscope signal 730. The spectrum 720 of the gyroscope signal 710 includes a peak 750, which corresponds to a peak 760 in spectrum 730 of the reference gyroscope signal 740. The frequency at the peak 750 corresponds to the respiratory rate.

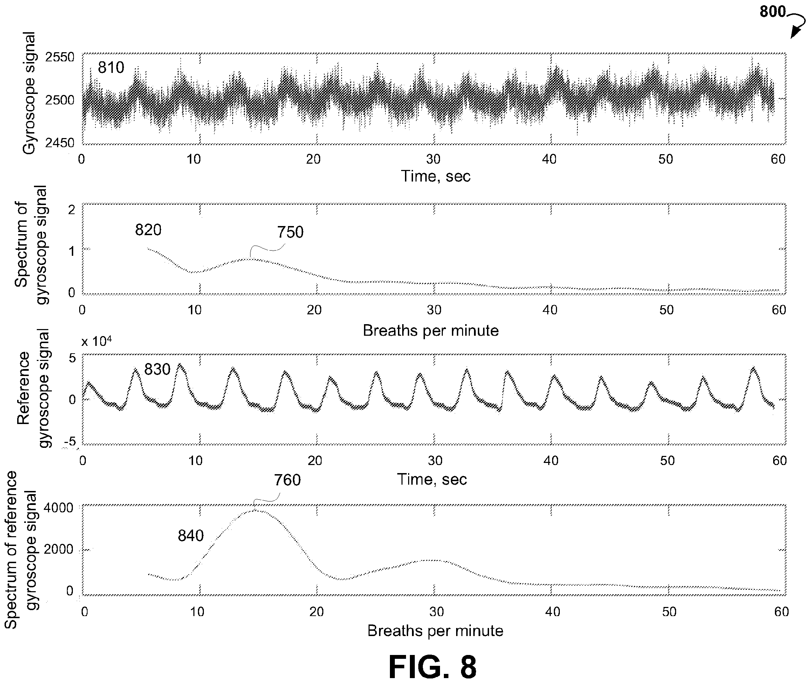

FIG. 8 shows plot 800 of example gyroscope signal 810 measured at a wrist, spectrum 820 of the gyroscope signal 810, reference gyroscope signal 830 measured at the chest, and spectrum 840 of the reference gyroscope signal 830. The SNR of gyroscope signal 810 is higher than SNR of the gyroscope signal 710 shown in FIG. 7. The spectrum 820 of the gyroscope signal 810 includes a peak 850 which corresponds to peak 860 in spectrum 830 of the reference gyroscope signal 840. The frequency at the peak 850 corresponds to the respiratory rate.

The respiratory rate estimation module 530 receives the gyroscope signal measured by the gyroscope 430 at the wrist of the patient and performs the spectral analysis of the gyroscope signal to obtain a spectrum of the gyroscope signal in a pre-determined range. The pre-determined range can be selected to cover a range of typical values for the respiratory rate of a human (for example, 6-18 breaths per minute). For example, the pre-determined range may include a range of 0 to 50 breaths per minute. In some embodiments, the spectral analysis may include fast Fourier transform, averaged periodograms (Bartlett's method), Welch's method, least-squares spectral analysis, and so forth.

The module 530 is further configured to determine a position of a peak in the spectrum that corresponds to the number of breaths of a patient per minute (the respiratory rate). In some embodiments, the module 530 is configured to select the strongest peak in the pre-determined range. As seen in FIG. 7 and FIG. 8, the spectrum of the gyroscope signal can be contaminated by a noise caused by movement of the patient. Typically, the noise is of a low frequency. Therefore, in the spectrum, the frequencies caused by the noise may interfere with the frequency corresponding to the respiratory rate.

The signal *s(t)* representing motion of breathing can be described by formula:

$$s(t) = \sum_{n=1}^{\infty} A_n f(n * \omega * t) \quad (4)$$

wherein *f* is a periodic function, $\omega$ is a fundamental frequency, and $A_n$ are descending amplitudes. In a frequency domain, the signal *s(t)* is represented by peaks at frequencies $n*\omega$, wherein amplitudes of the peaks descend with the increase of *n*. This pattern is searched in a spectrum of the gyroscope signal measured by the gyroscope at a wrist of patient to confirm that a correct peak was assigned to the respiratory rate.

After determining the strongest peak, the module 530 can confirm that the strongest peak in the spectrum represents breathing of the patient and not caused by movement noise of the patient. The module 530 is configured to determine that the spectrum includes one or more further peaks with descending amplitudes, such as the further peaks corresponding to frequencies $n*\omega$, wherein $\omega$ is a frequency corresponding to the strongest peak, and n is a natural number. Based on the determination, the module 530 assigns value $\omega$ to the value of the respiratory rate.

In some embodiments, the output module 540 is configured to provide reports and alert messages regarding a health status of the patient. The output module 540 may be configured to display via a graphical display system of the wearable device 110 or the mobile device 140. The output module 540 may determine that the respiratory rate of the patient is near or outside of a typical range (for example, 6-18 breaths per minute). The output module 540 may also determine that the value of oxygen saturation of the patient is below a pre-determined threshold. In response to the determination, the output module 540 may provide an alert message to the patient via a graphical display system of the wearable device 110 or/and mobile device 140. The alert message may include an advice to take medication or contact a doctor. The output module may also send a warning message, via the communication unit of the wearable device 110 and/or mobile device 140, to a computing device of a relative, a doctor, or a caretaker of the patient.

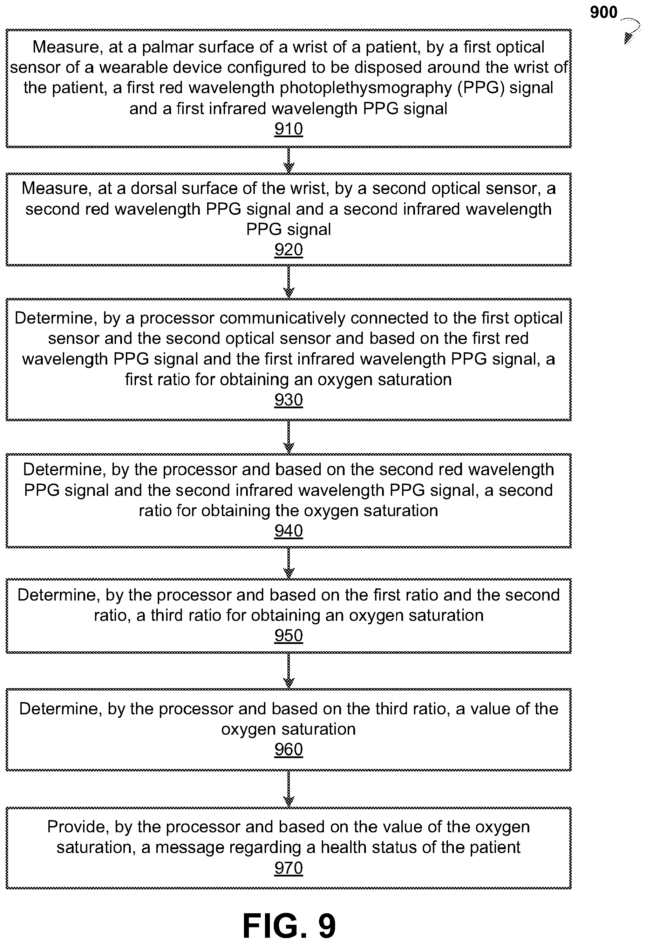

FIG. 9 is a flow chart diagram showing example method 900 for monitoring oxygen saturation of a patient. The method 900 may be implemented in system 100. In block 910, the method 900 may commence with measuring at a palmar surface of a wrist of the patient, by a first optical sensor, a first red wavelength PPG signal and a first infrared wavelength PPG signal. The first optical sensor can be embedded into a wearable device disposed around the wrist of the patient.

In block 920, the method 900 may proceed with measuring, at a dorsal surface of the wrist, by a second optical sensor disposed in the wearable device, a second red wavelength PPG signal and a second infrared wavelength PPG signal. The second red wavelength PPG signal and the second infrared wavelength PPG signal can be measured substantially simultaneously with the first red wavelength PPG signal and the first infrared wavelength PPG signal.

In block 930, the method 900 may continue with determining, by a processor communicatively connected to the first optical sensor and the second optical sensor and based on the first red wavelength PPG signal and the first infrared wavelength PPG signal, a first ratio for obtaining an oxygen saturation. In block 940, the method 900 may determine, by the processor and based on the second red wavelength PPG signal and the second infrared wavelength PPG signal, a second ratio for obtaining the oxygen saturation.

In block 950, the method 900 may determine, by the processor and based on the first ratio and the second ratio, a third ratio for obtaining an oxygen saturation. In block 960, the method 900 may determine, by the processor and based on the third ratio, a value of the oxygen saturation. In block 970, the method 900 may include providing, by the processor and based on the value of the oxygen saturation, a message regarding a health status of the patient. The message may include a warning against upcoming episode or worsening of a chronic disease of the patient, for which the oxygen saturation is one of the indicators. Upon receiving such a warning, preventive measures can be taken to reduce the severity of upcoming or worsening of the chronic disease. The message may include an advice to take a medicine or to contact a doctor.

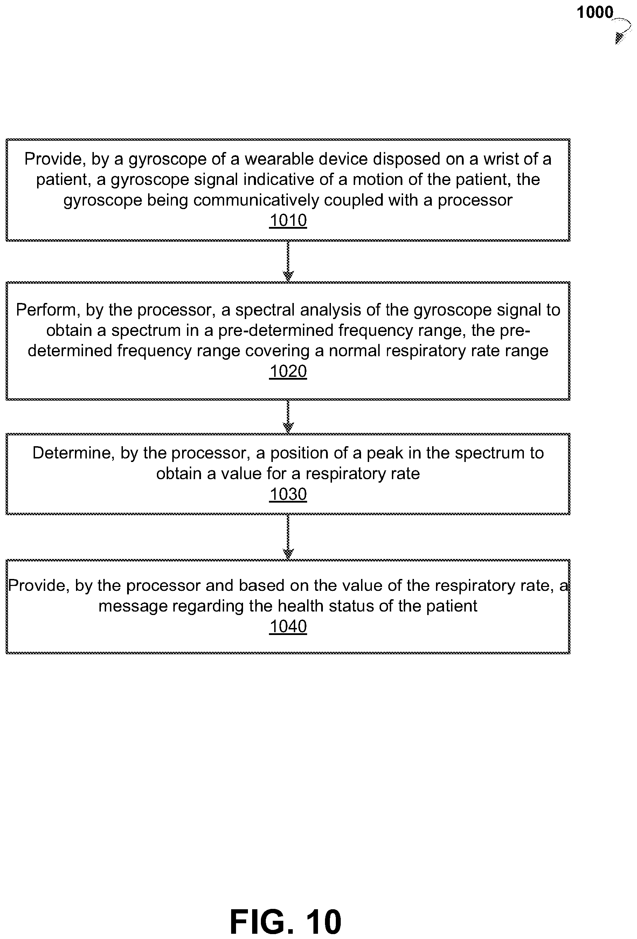

FIG. 10 is a flow chart diagram showing an example method 1000 for monitoring respiratory rate of a patient. The method 1000 may be implemented in system 100. In block 1010, the method 1000 may commence with providing, by a gyroscope of a wearable device disposed on a wrist of a patient, a gyroscope signal indicative of a motion of the patient. The gyroscope can be communicatively coupled with a processor. In block 1020, the method 1000 may proceed with performing, by the processor, a spectral analysis of the gyroscope signal to obtain a spectrum in a pre-determined frequency range, the pre-determined frequency range covering a normal respiratory rate range. In block 1030, the method 1000 may determine, by the processor, a position of a peak in the spectrum to obtain a value for a respiratory rate. In block 1040, the method 1000 may provide, by the processor and based on the value of the respiratory rate, a message regarding the health status of the patient. In some embodiments, the method 1000 may determine, by the processor, that the value of the respiratory rate is outside of the normal respiratory rate range and provide, by the processor, an alert message regarding the health status of the patient based on the determination that the value of the respiratory rate is outside of the normal respiratory rate range. The alert message may include a warning against upcoming or worsening of a chronic disease from which the patients suffers, for which respiratory rate is one of the indicators. Upon receiving the message, preventive measures can be taken to reduce the severity of upcoming or worsening of a chronic disease. The message may include an advice to take a medication or to contact a doctor.

The present technology is described above with reference to example embodiments. Therefore, other variations upon the example embodiments are intended to be covered by the present disclosure.

**Claims**

1. A system for monitoring a respiratory rate of a patient, the system comprising:

    a wearable device (110) configured to be disposed around a wrist of the patient, the wearable device (110) comprising a gyroscope (430) configured to provide (1010) a gyroscope signal (810) indicative of a motion of the patient; and
    a processor (220) communicatively connected to the gyroscope (430), the processor (220) being configured to:

        perform (1020) a spectral analysis of the gyroscope signal (810) to obtain a spectrum in a pre-determined range, the pre-determined range covering a normal respiratory rate range;
        determine (1030) a position of a strongest peak in the spectrum to obtain a value for the respiratory rate;
        determine that the spectrum includes one or more further peaks with descending amplitudes, the one or more further peaks corresponding to frequencies $n*\omega$, wherein $\omega$ is a frequency corresponding to the strongest peak, and n is a natural number;
        based on the determination, assign $\omega$ to the value of the respiratory rate; and
        provide (1040), based on the value of the respiratory rate, a message regarding a health status of the patient.

2. The system of claim 1, wherein:

    the wearable device (110) further comprises:

        a first optical sensor (222a) configured to measure, at a palmar surface of the wrist, a first red wavelength photoplethysmography (PPG) signal and a first infrared wavelength PPG signal; and

a second optical sensor (222b) configured to measure, at a dorsal surface of the wrist, a second red wavelength PPG signal and a second infrared wavelength PPG signal, wherein the first optical sensor and the second optical sensor are communicatively coupled with the processor; and

the processor is configured to:

determine, based on the first red wavelength PPG signal and the first infrared wavelength PPG signal, a first ratio for obtaining an oxygen saturation;

determine, based on the second red wavelength PPG signal and the second infrared wavelength PPG signal, a second ratio for obtaining oxygen saturation;

determine, based on the first ratio and the second ratio, a third ratio for obtaining an oxygen saturation;

determine, based on the third ratio, a value of the oxygen saturation; and

provide, based on the value of the oxygen saturation, a further message regarding the health status of the patient.

3. A method for monitoring a respiratory rate of a patient, the method comprising:

providing (1010), by a gyroscope (430) disposed in a wearable device (110), a gyroscope signal (810) indicative of a motion of the patient, wherein the wearable device (110) is configured to be worn around a wrist of the patient;

performing (1020), by a processor (220) communicatively coupled with the gyroscope (430), a spectral analysis of the gyroscope signal (810) to obtain a spectrum in a pre-determined range, the pre-determined range covering a normal respiratory rate range;

determining (1030), by the processor (220), a position of a strongest peak in the spectrum to obtain a value for the respiratory rate;

determining, by the processor (220), that the spectrum includes one or more further peaks with descending amplitudes, the one or more further peaks corresponding to frequencies n*$\omega$, wherein $\omega$ is a frequency corresponding to the strongest peak, and n is a natural number;

based on the determination that the spectrum includes one or more further peaks, assigning, by the processor (220), the $\omega$ to the respiratory rate; and

providing (1040), by the processor (220) and based on the value of the respiratory rate, a message regarding a health status of the patient.

4. The system of claim 1 or the method of claim 3, wherein the spectral analysis is performed by a method of averaged periodograms.

5. The system of claim 1 or the method of claim 3, wherein the normal respiratory rate range is 6 to 18 breaths per minute.

6. The method of claim 3, further comprising:

measuring (910), at a palmar surface of the wrist of the patient, by a first optical sensor (222a) of the wearable device (110), a first red wavelength photoplethysmography (PPG) signal and a first infrared wavelength PPG signal;

measuring (920), at a dorsal surface of the wrist, by a second optical sensor (222b) of the wearable device (110), a second red wavelength PPG signal and a second infrared wavelength PPG signal, the first optical sensor and the second optical sensor being communicatively coupled to the processor;

determining (930), by the processor and based on the first red wavelength PPG signal and the first infrared wavelength PPG signal, a first ratio for obtaining an oxygen saturation;

determining (940), by the processor and based on the second red wavelength PPG signal and the second infrared wavelength PPG signal, a second ratio for obtaining the oxygen saturation;

determining (950), by the processor and based on the first ratio and the second ratio, a third ratio for obtaining an oxygen saturation;

determining (960), by the processor and based on the third ratio, a value of the oxygen saturation; and

providing (970), by the processor and based on the value of the oxygen saturation, a further message regarding the health status of the patient.

7. The system of claim 2 or the method of claim 6, wherein the third ratio is determined by formula $R = \alpha R_a + (1- \alpha)R_b$, wherein the $\alpha$ is a weight between 0 and 1 and $R_a$ is the first ratio, and the $R_b$ is the second ratio.

8. The system or the method of claim 7, wherein:
the weight $\alpha$ is a function of perfusion index determined based on the second red wavelength PPG signal or the second infrared wavelength PPG signal.

9. The system or the method of claim 8, wherein the weight $\alpha$ increases when the perfusion index increases.

10. The system of claim 2 or the method of claim 6, wherein the first optical sensor (222a) is configured to measure the first red wavelength PPG signal and the first infrared wavelength PPG signal substantially near a radial artery of the wrist.

11. A non-transitory computer-readable storage medium having embodied thereon instructions, which when executed by a processor (220), perform steps of a method, the method comprising:

acquiring (1010) a gyroscope signal (810) indicative of a motion of a patient, the gyroscope signal (810) being measured by a gyroscope (430) disposed in a wearable device (110) configured to be worn around a wrist of the patient, communicatively coupled with the processor (220);
performing (1020) a spectral analysis of the gyroscope signal (810) to obtain a spectrum in a pre-determined range, the pre-determined range covering a normal respiratory rate range;
determining (1030) a position of a strongest peak in the spectrum to obtain a value for a respiratory rate;
determining that the spectrum includes one or more further peaks with descending amplitudes, the one or more further peaks corresponding to frequencies n*$\omega$, wherein $\omega$ is a frequency corresponding to the strongest peak, and n is a natural number;
based on the determination that the spectrum includes one or more further peaks, assigning the $\omega$ to the respiratory rate; and
providing (1040), based on the value of the respiratory rate, a message regarding a health status of the patient.

**Patentansprüche**

1. System zum Überwachen einer Atemfrequenz eines Patienten, das System umfassend:

eine tragbare Vorrichtung (110), die konfiguriert ist, um um ein Handgelenk des Patienten herum angeordnet zu sein, die tragbare Vorrichtung (110) umfassend ein Gyroskop (430), das konfiguriert ist, um ein Gyroskopsignal (810), das eine Bewegung des Patienten angibt, bereitzustellen (1010); und
einen Prozessor (220), der mit dem Gyroskop (430) kommunikativ verbunden ist, wobei der Prozessor (220) konfiguriert ist zum:

Durchführen (1020) einer Spektralanalyse des Gyroskopsignals (810), um ein Spektrum in einem zuvor bestimmten Bereich zu erhalten, wobei der zuvor bestimmte Bereich einen normalen Atemfrequenzbereich abdeckt;
Bestimmen (1030) einer Position eines stärksten Peaks in dem Spektrum, um einen Wert für die Atemfrequenz zu erhalten;
Bestimmen, dass das Spektrum einen oder mehrere weitere Peaks mit absteigenden Amplituden einschließt, wobei der eine oder die mehreren weiteren Peaks den Frequenzen n*$\omega$ entsprechen, wobei $\omega$ eine Frequenz, die dem stärksten Peak entspricht, ist und n eine natürliche Zahl ist;
basierend auf der Bestimmung, Zuordnen von $\omega$ zu dem Wert der Atemfrequenz; und
Bereitstellen (1040), basierend auf dem Wert der Atemfrequenz, einer Nachricht bezüglich eines Gesundheitszustands des Patienten.

2. System nach Anspruch 1, wobei:
die tragbare Vorrichtung (110) ferner umfasst:

einen ersten optischen Sensor (222a), der konfiguriert ist, um, an einer palmaren Oberfläche des Handgelenks, ein erstes Photoplethysmographiesignal (PPG-Signal) roter Wellenlänge und ein erstes PPG-Signal infraroter Wellenlänge zu messen; und
einen zweiten optischen Sensor (222b), der konfiguriert ist, um, an einer dorsalen Oberfläche des Handgelenks, ein zweites PPG-Signal roter Wellenlänge und ein zweites PPG-Signal infraroter Wellenlänge zu messen, wobei der erste optische Sensor und der zweite optische Sensor mit dem Prozessor kommunikativ gekoppelt sind; und

der Prozessor konfiguriert ist zum:

Bestimmen, basierend auf dem ersten PPG-Signal roter Wellenlänge und dem ersten PPG-Signal infraroter Wellenlänge, eines ersten Verhältnisses zum Erhalten einer Sauerstoffsättigung;

Bestimmen, basierend auf dem zweiten PPG-Signal roter Wellenlänge und dem zweiten PPG-Signal infraroter Wellenlänge, eines zweiten Verhältnisses zum Erhalten der Sauerstoffsättigung;

Bestimmen, basierend auf dem ersten Verhältnis und dem zweiten Verhältnis, eines dritten Verhältnisses zum Erhalten einer Sauerstoffsättigung;

Bestimmen, basierend auf dem dritten Verhältnis, eines Werts der Sauerstoffsättigung; und

Bereitstellen, basierend auf dem Wert der Sauerstoffsättigung, einer weiteren Nachricht bezüglich des Gesundheitszustands des Patienten.

3. Verfahren zum Überwachen einer Atemfrequenz eines Patienten, das Verfahren umfassend:

Bereitstellen (1010), durch ein Gyroskop (430), das in einer tragbaren Vorrichtung (110) angeordnet ist, eines Gyroskopsignals (810), das eine Bewegung des Patienten angibt, wobei die tragbare Vorrichtung (110) konfiguriert ist, um um ein Handgelenk des Patienten herum getragen zu werden;

Durchführen (1020), durch einen Prozessor (220), der mit dem Gyroskop (430) kommunikativ gekoppelt ist, einer Spektralanalyse des Gyroskopsignals (810), um ein Spektrum in einem zuvor bestimmten Bereich zu erhalten, wobei der zuvor bestimmte Bereich einen normalen Atemfrequenzbereich abdeckt;

Bestimmen (1030), durch den Prozessor (220), einer Position eines stärksten Peaks in dem Spektrum, um einen Wert für die Atemfrequenz zu erhalten;

Bestimmen, durch den Prozessor (220), dass das Spektrum einen oder mehrere weitere Peaks mit absteigenden Amplituden einschließt, wobei der eine oder die mehreren weiteren Peaks den Frequenzen n*ω entsprechen, wobei ω eine Frequenz, die dem stärksten Peak entspricht, ist und n eine natürliche Zahl ist;

basierend auf der Bestimmung, dass das Spektrum einen oder mehrere weitere Peaks einschließt, Zuordnen, durch den Prozessor (220), des ω zu der Atemfrequenz; und

Bereitstellen (1040), durch den Prozessor (220) und basierend auf dem Wert der Atemfrequenz, einer Nachricht bezüglich eines Gesundheitszustands des Patienten.

4. System nach Anspruch 1 oder Verfahren nach Anspruch 3, wobei die Spektralanalyse durch ein Verfahren von gemittelten Periodogrammen durchgeführt wird.

5. System nach Anspruch 1 oder Verfahren nach Anspruch 3, wobei der normale Atemfrequenzbereich 6 bis 18 Atemzüge pro Minute beträgt.

6. Verfahren nach Anspruch 3, ferner umfassend:

Messen (910), an einer palmaren Oberfläche des Handgelenks des Patienten, durch einen ersten optischen Sensor (222a) der tragbaren Vorrichtung (110), eines ersten Photoplethysmographiesignals (PPG-Signal) roter Wellenlänge und eines ersten PPG-Signals infraroter Wellenlänge;

Messen (920), an einer dorsalen Oberfläche des Handgelenks, durch einen zweiten optischen Sensor (222b) der tragbaren Vorrichtung (110), eines zweiten PPG-Signals roter Wellenlänge und eines zweiten PPG-Signals infraroter Wellenlänge, wobei der erste optische Sensor und der zweite optische Sensor mit dem Prozessor kommunikativ gekoppelt sind;

Bestimmen (930), durch den Prozessor und basierend auf dem ersten PPG-Signal roter Wellenlänge und dem ersten PPG-Signal infraroter Wellenlänge, eines ersten Verhältnisses zum Erhalten einer Sauerstoffsättigung;

Bestimmen (940), durch den Prozessor und basierend auf dem zweiten PPG-Signal roter Wellenlänge und dem zweiten PPG-Signal infraroter Wellenlänge, eines zweiten Verhältnisses zum Erhalten der Sauerstoffsättigung;

Bestimmen (950), durch den Prozessor und basierend auf dem ersten Verhältnis und dem zweiten Verhältnis, eines dritten Verhältnisses zum Erhalten einer Sauerstoffsättigung;

Bestimmen (960), durch den Prozessor und basierend auf dem dritten Verhältnis, eines Werts der Sauerstoffsättigung; und

Bereitstellen (970), durch den Prozessor und basierend auf dem Wert der Sauerstoffsättigung, einer weiteren Nachricht bezüglich des Gesundheitszustands des Patienten.

7. System nach Anspruch 2 oder Verfahren nach Anspruch 6, wobei das dritte Verhältnis durch Formel $R = a\,R_a + (1 - a)\,R_b$ bestimmt wird, wobei a ein Gewicht zwischen 0 und 1 ist und $R_a$ das erste Verhältnis ist und $R_b$ das zweite Verhältnis ist.

8. System oder Verfahren nach Anspruch 7, wobei:
das Gewicht a abhängig von einem Perfusionsindex, der basierend auf dem zweiten PPG-Signal roter Wellenlänge oder dem zweiten PPG-Signal infraroter Wellenlänge bestimmt wird, ist.

9. System oder Verfahren nach Anspruch 8, wobei das Gewicht a zunimmt, wenn der Perfusionsindex zunimmt.

10. System nach Anspruch 2 oder Verfahren nach Anspruch 6, wobei der erste optische Sensor (222a) konfiguriert ist, um das erste PPG-Signal roter Wellenlänge und das erste PPG-Signal infraroter Wellenlänge im Wesentlichen nahe einer Radialarterie des Handgelenks zu messen.

11. Nichtflüchtiges computerlesbares Speichermedium, das Anweisungen darauf integriert aufweist, die, wenn sie durch einen Prozessor (220) ausgeführt werden, Schritte eines Verfahrens durchführen, das Verfahren umfassend:

Erfassen (1010) eines Gyroskopsignals (810), das eine Bewegung eines Patienten angibt, wobei das Gyroskopsignal (810) durch ein Gyroskop (430) gemessen wird, das in einer tragbaren Vorrichtung (110) angeordnet ist, die konfiguriert ist, um um ein Handgelenk des Patienten herum getragen zu werden, das mit dem Prozessor (220) kommunikativ gekoppelt ist;
Durchführen (1020) einer Spektralanalyse des Gyroskopsignals (810), um ein Spektrum in einem zuvor bestimmten Bereich zu erhalten, wobei der zuvor bestimmte Bereich einen normalen Atemfrequenzbereich abdeckt;
Bestimmen (1030) einer Position eines stärksten Peaks in dem Spektrum, um einen Wert für eine Atemfrequenz zu erhalten;
Bestimmen, dass das Spektrum einen oder mehrere weitere Peaks mit absteigenden Amplituden einschließt, wobei der eine oder die mehreren weiteren Peaks den Frequenzen n*$\omega$ entsprechen, wobei $\omega$ eine Frequenz, die dem stärksten Peak entspricht, ist und n eine natürliche Zahl ist;
basierend auf der Bestimmung, dass das Spektrum einen oder mehrere weitere Peaks einschließt, Zuordnen des $\omega$ zu der Atemfrequenz; und
Bereitstellen (1040), basierend auf dem Wert der Atemfrequenz, einer Nachricht bezüglich eines Gesundheitszustands des Patienten.

**Revendications**

1. Système permettant de surveiller une fréquence respiratoire d'un patient, le système comprenant :

un dispositif portable (110) conçu pour être disposé autour d'un poignet du patient, le dispositif portable (110) comprenant un gyroscope (430) configuré pour fournir (1010) un signal gyroscopique (810) indiquant un mouvement du patient ; et
un processeur (220) couplé en communication au gyroscope (430), le processeur (220) étant configuré pour :

réaliser (1020) une analyse spectrale du signal gyroscopique (810) pour obtenir un spectre dans une plage prédéterminée, la plage prédéterminée couvrant une plage de fréquences respiratoires normale ;
déterminer (1030) une position d'un pic le plus fort dans le spectre pour obtenir une valeur pour la fréquence respiratoire ;
déterminer que le spectre comporte un ou plusieurs pics supplémentaires avec des amplitudes descendantes, le ou les pics supplémentaires correspondant à des n*$\omega$ fréquences, où $\omega$ est une fréquence correspondant au pic le plus fort, et n est un nombre naturel ;
sur la base de la détermination, attribuer $\omega$ à la valeur de la fréquence respiratoire ; et
fournir (1040), sur la base de la valeur de la fréquence respiratoire, un message concernant un état de santé du patient.

2. Système selon la revendication 1, dans lequel :
le dispositif portable (110) comprend en outre :

un premier capteur optique (222a) configuré pour mesurer, au niveau d'une surface palmaire du poignet, un premier signal de photopléthysmographie (PPG) à longueur d'onde rouge et un premier signal PPG à longueur d'onde infrarouge ; et

un second capteur optique (222b) configuré pour mesurer, au niveau d'une surface dorsale du poignet, un second signal PPG à longueur d'onde rouge et un second signal PPG à longueur d'onde infrarouge, dans lequel le premier capteur optique et le second capteur optique sont couplés en communication avec le processeur ; et

le processeur étant configuré pour :

déterminer, sur la base du premier signal PPG à longueur d'onde rouge et du premier signal PPG à longueur d'onde infrarouge, un premier rapport pour obtenir une saturation en oxygène ;

déterminer, sur la base du second signal PPG à longueur d'onde rouge et du second signal PPG à longueur d'onde infrarouge, un deuxième rapport pour obtenir une saturation en oxygène ;

déterminer, sur la base du premier rapport et du deuxième rapport, un troisième rapport pour obtenir une saturation en oxygène ;

déterminer, sur la base du troisième rapport, une valeur de la saturation en oxygène ; et

fournir, sur la base de la valeur de la saturation en oxygène, un autre message concernant l'état de santé du patient.

3. Procédé de surveillance d'une fréquence respiratoire d'un patient, le procédé comprenant :

la fourniture (1010), par un gyroscope (430) disposé dans un dispositif portable (110), d'un signal gyroscopique (810) indiquant un mouvement du patient, dans lequel le dispositif portable (110) est conçu pour être porté autour d'un poignet du patient ;

la réalisation (1020), par un processeur (220) couplé en communication au gyroscope (430), d'une analyse spectrale du signal gyroscopique (810) pour obtenir un spectre dans une plage prédéterminée, la plage prédéterminée couvrant une plage de fréquences respiratoires normale ;

la détermination (1030), par le processeur (220), d'une position d'un pic le plus fort dans le spectre pour obtenir une valeur pour la fréquence respiratoire ;

la détermination, par le processeur (220), du fait que le spectre comporte un ou plusieurs pics supplémentaires avec des amplitudes descendantes, le ou les pics supplémentaires correspondant à des $n*\omega$ fréquences, où $\omega$ est une fréquence correspondant au pic le plus fort, et n est un nombre naturel ;

sur la base de la détermination du fait que le spectre comporte un ou plusieurs pics supplémentaires, l'attribution, par le processeur (220), de $\omega$ à la fréquence respiratoire ; et

la fourniture (1040), par le processeur (220) et sur la base de la valeur de la fréquence respiratoire, d'un message concernant un état de santé du patient.

4. Système selon la revendication 1 ou procédé selon la revendication 3, dans lequel l'analyse spectrale est réalisée par un procédé de périodogrammes moyennés.

5. Système selon la revendication 1 ou procédé selon la revendication 3, dans lequel la plage de fréquences respiratoires normale se situe entre 6 et 18 respirations par minute.

6. Procédé selon la revendication 3, comprenant en outre :

la mesure(910), au niveau d'une surface palmaire du poignet du patient, par un premier capteur optique (222a) du dispositif portable (110), d'un premier signal de photopléthysmographie (PPG) à longueur d'onde rouge et d'un premier signal PPG à longueur d'onde infrarouge ;

la mesure (920), au niveau d'une surface dorsale du poignet, par un second capteur optique (222b) du dispositif portable (110), d'un second signal PPG à longueur d'onde rouge et d'un second signal PPG à longueur d'onde infrarouge, le premier capteur optique et le second capteur optique étant couplés en communication au processeur ;

la détermination (930), par le processeur et sur la base du premier signal PPG à longueur d'onde rouge et du premier signal PPG à longueur d'onde infrarouge, d'un premier rapport pour obtenir une saturation en oxygène ;

la détermination (940), par le processeur et sur la base du second signal PPG à longueur d'onde rouge et du second signal PPG à longueur d'onde infrarouge, d'un deuxième rapport pour obtenir la saturation en oxygène ;

la détermination (950), par le processeur et sur la base du premier rapport et du deuxième rapport, d'un troisième rapport pour obtenir une saturation en oxygène ;

la détermination (960), par le processeur et sur la base du troisième rapport, d'une valeur de la saturation en oxygène ; et

la fourniture (970), par le processeur et sur la base de la valeur de la saturation en oxygène, d'un autre message concernant l'état de santé du patient.

7. Système selon la revendication 2 ou procédé selon la revendication 6, dans lequel le troisième rapport est déterminé par la formule $R = a\,R_a + (1 - \alpha)R_b$, dans lequel a est un poids compris entre 0 et 1 et $R_a$ est le premier rapport, et $R_b$ est le deuxième rapport.

8. Système ou procédé selon la revendication 7, dans lequel :
le poids a est une fonction d'indice de perfusion déterminée à partir du second signal PPG à longueur d'onde rouge ou du second signal PPG à longueur d'onde infrarouge.

9. Système ou procédé selon la revendication 8, dans lequel le poids a augmente lorsque l'indice de perfusion augmente.

10. Système selon la revendication 2 ou procédé selon la revendication 6, dans lequel le premier capteur optique (222a) est configuré pour mesurer le premier signal PPG à longueur d'onde rouge et le premier signal PPG à longueur d'onde infrarouge sensiblement près d'une artère radiale du poignet.

11. Support de stockage non transitoire lisible par ordinateur sur lequel sont intégrées des instructions qui, lorsqu'elles sont exécutées par un processeur (220), réalisent des étapes d'un procédé, le procédé comprenant :

l'acquisition (1010) d'un signal gyroscopique (810) indiquant un mouvement d'un patient, le signal gyroscopique (810) étant mesuré par un gyroscope (430) disposé dans un dispositif portable (110) conçu pour être porté autour d'un poignet du patient, couplé de manière communicative au processeur (220) ;
la réalisation (1020) d'une analyse spectrale du signal gyroscopique (810) pour obtenir un spectre dans une plage prédéterminée, la plage prédéterminée couvrant une plage de fréquences respiratoires normale ;
la détermination (1030) d'une position d'un pic le plus fort dans le spectre pour obtenir une valeur pour une fréquence respiratoire ;
la détermination du fait que le spectre comporte un ou plusieurs pics supplémentaires avec des amplitudes descendantes, le ou les pics supplémentaires correspondant à des n*ω fréquences, où ω est une fréquence correspondant au pic le plus fort, et n est un nombre naturel ;
sur la base de la détermination du fait que le spectre comporte un ou plusieurs pics supplémentaires, l'attribution de ω à la fréquence respiratoire ; et
la fourniture (1040), sur la base de la valeur de la fréquence respiratoire, d'un message concernant un état de santé du patient.

FIG. 1

EP 3 849 407 B1

FIG. 2

| Sensors 120 | Medical Parameters 310 | Chronic Diseases 320 |
|---|---|---|
| **Optical Sensors** 222a and 222b | Oxygen Saturation Cardiac Output Vascular Resistance Pulse Rate Blood pressure | CHF Hypertension Arrhythmia Asthma COPD Hypoglycemia Sleep Apnea Parkinson's Disease |
| **Electrical Sensors** 224 | ECG Respiration | |
| **Motion Sensors** 226 | Body movement Tremor | |

# FIG. 3

FIG. 4B

FIG. 4A

FIG. 4C

500

Sensor Data
Acquisition
Module
510

Oxygen Saturation
Estimation Module
520

Respiratory Rate
Estimation Module
530

Output Module
540

# FIG. 5

Light received by
photodetector

**600A**

610

Difference in the amount of light
620

Total amount of light received
630

Time

# FIG. 6A

**600B**

$\alpha$

1    2    3    4

Perfusion Index, %

# FIG. 6B

**FIG. 7**

FIG. 8

900

Measure, at a palmar surface of a wrist of a patient, by a first optical sensor of a wearable device configured to be disposed around the wrist of the patient, a first red wavelength photoplethysmography (PPG) signal and a first infrared wavelength PPG signal
910

Measure, at a dorsal surface of the wrist, by a second optical sensor, a second red wavelength PPG signal and a second infrared wavelength PPG signal
920

Determine, by a processor communicatively connected to the first optical sensor and the second optical sensor and based on the first red wavelength PPG signal and the first infrared wavelength PPG signal, a first ratio for obtaining an oxygen saturation
930

Determine, by the processor and based on the second red wavelength PPG signal and the second infrared wavelength PPG signal, a second ratio for obtaining the oxygen saturation
940

Determine, by the processor and based on the first ratio and the second ratio, a third ratio for obtaining an oxygen saturation
950

Determine, by the processor and based on the third ratio, a value of the oxygen saturation
960

Provide, by the processor and based on the value of the oxygen saturation, a message regarding a health status of the patient
970

# FIG. 9

**1000**

Provide, by a gyroscope of a wearable device disposed on a wrist of a patient, a gyroscope signal indicative of a motion of the patient, the gyroscope being communicatively coupled with a processor
1010

Perform, by the processor, a spectral analysis of the gyroscope signal to obtain a spectrum in a pre-determined frequency range, the pre-determined frequency range covering a normal respiratory rate range
1020

Determine, by the processor, a position of a peak in the spectrum to obtain a value for a respiratory rate
1030

Provide, by the processor and based on the value of the respiratory rate, a message regarding the health status of the patient
1040

# FIG. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 73866615
- US 73863615
- US 73871115
- US 98311815
- US 983118
- WO 2017079828 A1
- WO 2013061415 A1
- US 2016007935 A1
- WO 2017140696 A1
- US 2018132794 A1